# EUROPEAN PATENT APPLICATION

(11) **EP 4 796 051 A1**
(43) Date of publication of application: **26.08.2026**
(21) Application number: 25791520.7
(22) Date of filing: 20.08.2025
(51) Int. Cl.: A61B 17/04

(54) **SUTURE ANCHOR STORAGE MODULE AND SEPARABLE MODULAR SUTURE ANCHOR IMPLANTATION SYSTEM**

(30) Priority: 02.01.2025 CN 202510000617
(71) Applicant: STAR SPORTS MEDICINE CO., LTD., Beijing 100176 (CN)
(72) Inventor: DONG, Wenxing, Beijing 100176 (CN); ZHANG, Zhiliang, Beijing 100176 (CN); YANG, Tengfei, Beijing 100176 (CN); YIN, Jichen, Beijing 100176 (CN)
(74) Representative: Angerhausen, Christoph
(86) International application number: PCT/CN2025/115775
(87) International publication number: WO 2026/144192

(57) **Abstract**

The present application discloses a suture anchor storage module and a separable modular suture anchor implant system, relating to the technical field of medical instruments. The suture anchor storage module includes an anchor, a suture, a carrier seat, and a winding and tensioning assembly. The suture is threaded through the anchor. The carrier seat is internally provided with an insertion cavity and a clamping hole, and the insertion cavity extends through two opposite ends of the carrier seat and allows an inserter to pass through; the clamping hole is in communication with the insertion cavity to clamp the anchor; and a mounting shaft is provided on a side of the carrier seat. The winding and tensioning assembly is rotatably connected to the mounting shaft, the suture is wound on the winding and tensioning assembly, and the winding and tensioning assembly rotates to release the suture.

## Description

### CROSS REFERENCE TO RELATED APPLICATION

This application claims priority to Chinese Patent Application No. 2025100006171, filed on January 2, 2025 and entitled "SUTURE ANCHOR STORAGE MODULE AND SEPARABLE MODULAR SUTURE ANCHOR IMPLANT SYSTEM", which is hereby incorporated by reference in its entirety.

### TECHNICAL FIELD

The present application relates to the technical field of medical instruments, in particular to a suture anchor storage module and a separable modular suture anchor implant system.

### BACKGROUND

Anchors are widely used for the fixation of bones and soft tissues. Soft tissues (such as tendons and ligaments) can be simply and effectively fixed to bones through anchors and sutures around shoulders, knees, hips, elbows, wrists, ankles, etc., without loosening or excessive tension until healing.

In an existing bone anchor implant system, an anchor and a suture are assembled on an inserter for storage. During surgery, the inserter is used with the anchor and the suture as one set, leading to direct discard of the inserter after surgery. In practice, the quantity and type of anchors are selected according to actual needs, leading to discard of a plurality of inserters in one surgery. The inserters cannot be reused, which not only wastes medical resources and pollutes the environment, but is not conducive to preoperative material preparation and surgical operations.

### SUMMARY

One of the objects of the present application is to provide a suture anchor storage module and a separable modular suture anchor implant system, to achieve separate storage of an anchor and a suture, avoid waste of inserters, and facilitate preoperative material preparation.

To achieve this object, the present application adopts the following technical solutions.

A suture anchor storage module includes: an anchor; a suture threaded through the anchor; a carrier seat, where the carrier seat is internally provided with an insertion cavity and a clamping hole, and the insertion cavity is disposed through two opposite ends of the carrier seat and is for allowing an inserter to pass through; the clamping hole is in communication with the insertion cavity to clamp the anchor; and a mounting shaft is provided on a side of the carrier seat; and a winding and tensioning assembly, wherein the winding and tensioning assembly is rotatably connected to the mounting shaft, the suture is wound on the winding and tensioning assembly, and the winding and tensioning assembly is configured to rotate to release the suture.

As an optional embodiment of the suture anchor storage module, the winding and tensioning assembly includes a winding reel and a damping member, the winding reel is sleeved on the mounting shaft and rotatably connected to the mounting shaft, the bottom of the winding reel is provided with a concave cavity, the circumference of the concave cavity is configured as an internal ratchet structure, the damping member is fixed on the side of the carrier seat and located in the internal ratchet structure, the damping member includes an elastic pawl, the winding reel is configured to rotate, and each of the elastic pawls is configured to engage with the internal ratchet structure to provide rotational resistance to the winding reel.

As an optional embodiment of the suture anchor storage module, the damping member further includes a mounting ring, and the mounting ring is sleeved on the mounting shaft and fixedly connected to the carrier seat; a plurality of the elastic pawls are provided at intervals on the periphery of the mounting ring, one end of each of the elastic pawls is connected to the mounting ring, the other end of each of of the elastic pawls is bent into a clamping ring, the clamping rings are spaced apart from the periphery of the mounting ring, a free end of the clamping ring is provided with a clamping protrusion, and the clamping protrusion elastically abuts against the internal ratchet structure.

As an optional embodiment of the suture anchor storage module, the winding reel includes an inner cylinder and an outer cylinder arranged coaxially, the inner cylinder is sleeved on the mounting shaft, the outer cylinder is provided with opposite suture retaining slots, the suture is wound on the outer cylinder, and a tail end of the suture is fixed by passing through the opposite suture retaining slots.

As an optional embodiment of the suture anchor storage module, the winding and tensioning assembly further includes a compression member, the compression member includes a gland and a connection column, the mounting shaft is provided with a central hole, the connection column is engaged with the central hole, and the winding reel is rotatably arranged between the gland and the carrier seat.

As an optional embodiment of the suture anchor storage module, a limit top plate and a limit bottom plate are provided at two ends of the winding reel respectively, the gland is in limiting engagement with the limit top plate, and the concave cavity is arranged on the side of the limit bottom plate away from the limit top plate.

As an optional embodiment of the suture anchor storage module, a mounting groove is provided on the side of the carrier seat, the mounting shaft is arranged in the mounting groove, the bottom of the mounting groove is provided with a retaining groove, the bottom of the damping member is provided with a clamping block, and the clamping block is engaged with the retaining groove.

As an optional embodiment of the suture anchor storage module, two winding and tensioning assemblies are provided, two winding and tensioning assemblies are symmetrically arranged on two sides of the carrier seat, one end of the suture passing through the anchor is wound on one of two winding and tensioning assembly, and the other end of the suture is wound on the other one of two winding and tensioning assembly;

or one winding and tensioning assembly is provided, the winding and tensioning assembly is arranged on one side of the carrier seat, and two ends of the suture passing through the anchor are each wound on the winding and tensioning assembly.

As an optional embodiment of the suture anchor storage module, the suture anchor storage module further includes a clamping assembly, and the clamping assembly is connected to the carrier seat; the clamping assembly includes a first clamping member and a second clamping member arranged symmetrically, and the first clamping member and the second clamping member are oppositely disposed to form a clamping hole.

As an optional embodiment of the suture anchor storage module, the clamping assembly further includes an elastic member, one elastic member is provided, and the elastic member connects the first clamping member and the second clamping member;

or two elastic members comprising a first elastic member and a second elastic member, are provided, the first elastic member is arranged at the end of the first clamping member away from the second clamping member, and the first elastic member is configured to elastically connect the first clamping member to the carrier seat; the second elastic member is arranged at the end of the second clamping member away from the first clamping member; and the second elastic member is configured to elastically connect the second clamping member to the carrier seat.

As an optional embodiment of the suture anchor storage module, both the first clamping member and the second clamping member are slidably arranged in the carrier seat; a sliding groove is provided in the carrier seat, a sliding column is provided on each of the first clamping member and the second clamping member, the sliding column is engaged with the sliding groove, and the sliding groove extends along an elastic expansion direction of the elastic member.

As an optional embodiment of the suture anchor storage module, two insertion cavities and two clamping holes are provided, and two insertion cavities and two clamping holes are correspondingly arranged.

As an optional embodiment of the suture anchor storage module, the end of the carrier seat away from the clamping hole is configured as a clamping end, and the clamping end is provided with a first fixing structure.

As an optional embodiment of the suture anchor storage module, the first fixing structure includes fixing grooves, and the fixing grooves are arranged on two opposite sides of the clamping end;

or the first fixing structure includes a fixing nail, the fixing nail is arranged at the end of the clamping end away from the clamping hole, and an end of the fixing nail is provided with a hook.

A separable modular suture anchor implant system includes an inserter and the suture anchor storage module as described in any of the above embodiments, wherein after the inserter is inserted into the insertion cavity and then into the anchor and pushes the anchor out of the carrier seat, the inserter is fixed to the carrier seat.

As an optional embodiment of the separable modular suture anchor implant system, the inserter includes a handle and an insertion rod, the insertion rod is connected to the handle, the end of the insertion rod away from the handle is provided with an insertion portion, and the insertion portion is in insertion engagement with the anchor.

As an optional embodiment of the separable modular suture anchor implant system, the end of the handle close to the insertion rod is provided with a clamping groove, and the clamping groove is provided with a second fixing structure.

As an optional embodiment of the separable modular anchoring system, the second fixing structure includes connection holes, and the connection holes are arranged on two opposite side walls of the clamping groove;

or the second fixing structure includes an insertion hole and a fixing groove, the insertion hole is arranged at the bottom of the clamping groove, and the fixing groove is arranged at the end of the insertion hole away from the clamping groove.

Beneficial effects of the present application are as follows:

According to the suture anchor storage module provided in the present application, both the anchor and the suture are stored on the carrier seat, and the carrier seat is internally provided with the insertion cavity allowing the inserter to pass through and the clamping hole for clamping the anchor. The mounting shaft is provided on the side of the carrier seat, the winding and tensioning assembly is rotatably connected to the mounting shaft, and the suture passes through the anchor and is wound on the winding and tensioning assembly. The inserter passing through the insertion cavity can be inserted into the anchor and pushes the anchor out of the carrier seat. The suture anchor storage module achieves separate storage of the anchor and the suture by designing the carrier seat to store the anchor and the suture, thereby facilitating preoperative material preparation. The suture anchor storage module cooperates with the inserter during surgery to achieve the implantation of the anchor and the suture; the winding and tensioning assembly enables the suture to be actively released under the tension of the anchor and maintain a tight state in the release process, thereby ensuring orderly release of the suture without the risk of entanglement, avoiding manual release of the suture by a doctor or additional adjustment, simplifying surgical operation steps, and facilitating surgical operations.

The separable modular suture anchor implant system provided in the present application, including the foregoing suture anchor storage module, achieves separate storage of the anchor and the suture and reuse of the inserter, facilitates preoperative material preparation, ensures orderly release of the suture without the risk of entanglement, avoids manual release of the suture by a doctor or additional adjustment, simplifies surgical operation steps, and reduces waste of medical resources and environmental pollution.

### BRIEF DESCRIPTION OF THE DRAWINGS

FIG. 1 is a schematic structural diagram of a suture anchor storage module according to a first embodiment of the present application;
FIG. 2 is a cross-sectional view of the suture anchor storage module according to the first embodiment of the present application;
FIG. 3 is a schematic structural diagram of a winding and tensioning assembly cooperating with an anchor and a suture according to the first embodiment of the present application;
FIG. 4 is a longitudinal cross-sectional view of a carrier seat according to the first embodiment of the present application;
FIG. 5 is a schematic diagram of a first structure of the carrier seat according to the first embodiment of the present application;
FIG. 6 is a schematic diagram of a second structure of the carrier seat according to the first embodiment of the present application;
FIG. 7 is an exploded schematic diagram of the winding and tensioning assembly according to the first embodiment of the present application;
FIG. 8 is a schematic structural diagram of a damping member cooperating with an internal ratchet structure at the bottom of a winding reel according to the first embodiment of the present application;
FIG. 9 is an exploded schematic diagram of a handle of an inserter of a separable modular suture anchor implant system and the suture anchor storage module according to the first embodiment of the present application;
FIG. 10 is an assembly diagram of the handle of the inserter of the separable modular suture anchor implant system and the suture anchor storage module according to the first embodiment of the present application;
FIG. 11 is a schematic structural diagram of a suture anchor storage module according to a second embodiment of the present application;
FIG. 12 is a schematic structural diagram of the suture anchor storage module according to the second embodiment of the present application, with a carrier seat omitted;
FIG. 13 is a schematic structural diagram of a carrier seat according to the second embodiment of the present application;
FIG. 14 is a longitudinal cross-sectional view of the carrier seat according to the second embodiment of the present application;
FIG. 15 is a top view of the carrier seat according to the second embodiment of the present application;
FIG. 16 is a schematic structural diagram of a handle of an inserter according to the second embodiment of the present application;
FIG. 17 is a schematic structural diagram of a separable modular suture anchor implant system according to the second embodiment of the present application;
FIG. 18 is a longitudinal cross-sectional view of the separable modular suture anchor implant system according to the second embodiment of the present application;
FIG. 19 is a schematic structural diagram of a suture anchor storage module storing one anchor according to a third embodiment of the present application;
FIG. 20 is a schematic structural diagram of the suture anchor storage module storing one anchor according to the third embodiment of the present application, with an upper cover omitted;
FIG. 21 is a schematic structural diagram of the suture anchor storage module storing one anchor according to the third embodiment of the present application, with an upper cover and a clamping assembly omitted;
FIG. 22 is a schematic structural diagram of a suture anchor storage module storing two anchors according to the third embodiment of the present application;
FIG. 23 is an exploded schematic diagram of the suture anchor storage module storing two anchors according to the third embodiment of the present application, with anchors and sutures omitted;
FIG. 24 is a schematic structural diagram of a carrier seat according to the third embodiment of the present application;
FIG. 25 is a schematic structural diagram of an upper cover according to the third embodiment of the present application;
FIG. 26 is a top view of the suture anchor storage module storing two anchors according to the third embodiment of the present application;
FIG. 27 is a cross-sectional view taken along a line A-A in FIG. 26;
FIG. 28 is a rear view of the suture anchor storage module storing two anchors according to the third embodiment of the present application; and
FIG. 29 is a cross-sectional view taken along a line B-B in FIG. 28.

### Reference numerals:

1. Anchor;
2. Suture;
3. Carrier seat; 31. Insertion cavity; 311. Guide slot; 32. Mounting groove; 321. Mounting shaft; 3211. Central hole; 322. Retaining groove; 331. First mounting cavity; 332. Second mounting cavity; 34. Clamping end; 341. Fixing groove; 342. Fixing nail; 3421. Hook; 35. Extension slot; 36. Communication cavity; 37. Mounting bottom shell; 371. Guide block; 3711. Through hole; 3712. Guide groove; 3713. Guide inclined surface; 3714. Blocking bar; 372. Suture collection groove; 373. Bayonet; 38. Upper cover; 381. Clamping bar; 39. Mounting cylinder; 391. Passing groove;
301. First sliding groove; 302. Second sliding groove;
4. Winding and tensioning assembly; 41. Winding reel; 411. Inner cylinder; 412. Outer cylinder; 4121. Suture retaining slot; 413. Limit top plate; 414. Limit bottom plate; 4141. Concave cavity; 4142. Internal ratchet structure; 42. Damping member; 421. Mounting ring; 4211. Clamping block; 422. Elastic pawl; 4221. Clamping ring; 4222. Clamping protrusion; 43. Compression member; 431. Gland; 432. Connection column; 4321. Protrusion;
5. Clamping assembly; 51. First clamping member; 511. First sliding column; 52. Second clamping member; 521. Second sliding column; 531. First elastic member; 532. Second elastic member; 54. Clamping hole;
6. Inserter; 61. Handle; 611. Clamping groove; 6111. Connection hole; 6112. Insertion hole; 6113. Fixing groove; 62. Insertion rod; 621. Insertion portion; 622. Guide column.

### DETAILED DESCRIPTION

Embodiments of the present application are described in detail below, and examples of the embodiments are shown in the accompanying drawings. The same or similar reference numerals throughout represent the same or similar elements or elements with the same or similar functions. The embodiments described below with reference to the accompanying drawings are exemplary, and are intended to explain the present application, but shall not be understood as limitations on the present application.

In the description of the present application, it should be noted that the orientations or positional relationships indicated by the terms "center", "upper", "lower", "left", "right", "vertical", "horizontal", "inner", "outer", etc. are based on the orientations or positional relationships shown in the accompanying drawings, and are intended to facilitate the description of the present application and simplify the description only, rather than indicating or implying that the device or element referred to must have a particular orientation or be constructed and operated in a particular orientation. Therefore, these terms cannot be interpreted as limiting the present application. In addition, the terms "first" and "second" are merely for description, and cannot be interpreted as indicating or implying relative importance. The terms "first position" and "second position" refer to two different positions.

Unless otherwise specified and limited, the terms "mounted", "connected", "connection", and "fixed" should be understood in a broad sense. For example, the "connection" may be fixed connection, detachable connection, mechanical connection, electrical connection, direct connection, indirect connection by a medium, internal communication of two elements, or interaction between two elements. A person of ordinary skill in the art may understand the specific meanings of the above terms in the present application according to specific circumstances.

Unless otherwise specified and limited, the first feature "above" or "below" the second feature may include direct contact between the first feature and the second feature, or may include contact between the first feature and the second feature through another feature between them instead of direct contact. Moreover, the first feature "on", "above", and "up" the second feature may include the first feature right above or obliquely above the second feature, or merely indicates that the level of the first feature is higher than that of the second feature. The first feature "below", "under", and "down" the second feature may include the first feature right below or obliquely below the second feature, or merely indicates that the level of the first feature is lower than that of the second feature.

The technical solution of the present application will be further explained through specific implementations in conjunction with the accompanying drawings.

### First embodiment:

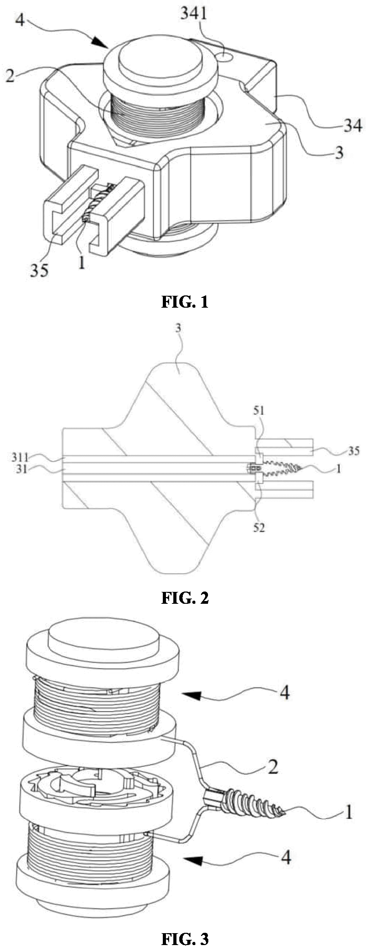

As shown in FIG. 1, FIG. 10, and FIG. 17, this embodiment provides a separable modular suture anchor implant system, including an inserter 6 and a suture anchor storage module. The inserter 6 and the suture anchor storage module are arranged separately, and an anchor 1 and a suture 2 are stored in the suture anchor storage module. The inserter 6 can be inserted into the suture anchor storage module and then into the anchor 1, and pushes out the anchor 1 from the suture anchor storage module to complete the assembly of the anchor 1 and the inserter 6 for subsequent implantation surgery of the anchor 1. After surgery, the suture 2 may be actively released. The separable modular suture anchor implant system achieves separate storage of the anchor 1 and the suture 2 and reuse of the inserter 6, avoids waste of the inserter 6, and facilitates preoperative material preparation. Meanwhile, the suture 2 is actively released from the suture anchor storage module after surgery, without manual release by a doctor, thereby simplifying surgical operations.

As shown in FIG. 1 to FIG. 6, this embodiment provides a suture anchor storage module, applied to the foregoing separable modular suture anchor implant system. The suture anchor storage module includes an anchor 1, a suture 2, a carrier seat 3, and a winding and tensioning assembly 4; the carrier seat 3 is internally provided with an insertion cavity 31 and a clamping hole 54, and the insertion cavity 31 extends through two opposite ends of the carrier seat 3 and allows an inserter 6 to pass through; the clamping hole 54 is in communication with the insertion cavity 31 to clamp the anchor 1; the suture 2 is threaded through the anchor 1, and a mounting shaft 321 is provided on a side of the carrier seat 3. The winding and tensioning assembly 4 is rotatably connected to the mounting shaft 321, the suture 2 is wound on the winding and tensioning assembly 4, and the winding and tensioning assembly 4 rotates to release the suture 2.

According to the suture anchor storage module provided in this embodiment, both the anchor 1 and the suture 2 are stored on the carrier seat 3, and the carrier seat 3 is internally provided with the insertion cavity 31 allowing the inserter 6 to pass through and the clamping hole 54 for clamping the anchor 1. The mounting shaft 321 is provided on the side of the carrier seat 3, the winding and tensioning assembly 4 is rotatably connected to the mounting shaft 321, and the suture 2 passes through the anchor 1 and is wound on the winding and tensioning assembly 4. The inserter 6 passing through the insertion cavity 31 can be inserted into the anchor 1 and pushes the anchor 1 out of the carrier seat 3. The suture anchor storage module achieves separate storage of the anchor 1 and the suture 2 by designing the carrier seat 3 to store the anchor 1 and the suture 2, thereby facilitating preoperative material preparation. The suture anchor storage module cooperates with the inserter 6 during surgery to achieve the implantation of the anchor 1 and the suture 2; the winding and tensioning assembly 4 enables the suture 2 to be actively released under the tension of the anchor 1 and maintain a tight state in the release process, thereby ensuring orderly release of the suture 2 without the risk of entanglement, avoiding manual release of the suture 2 by a doctor or additional adjustment, simplifying surgical operation steps, and facilitating surgical operations.

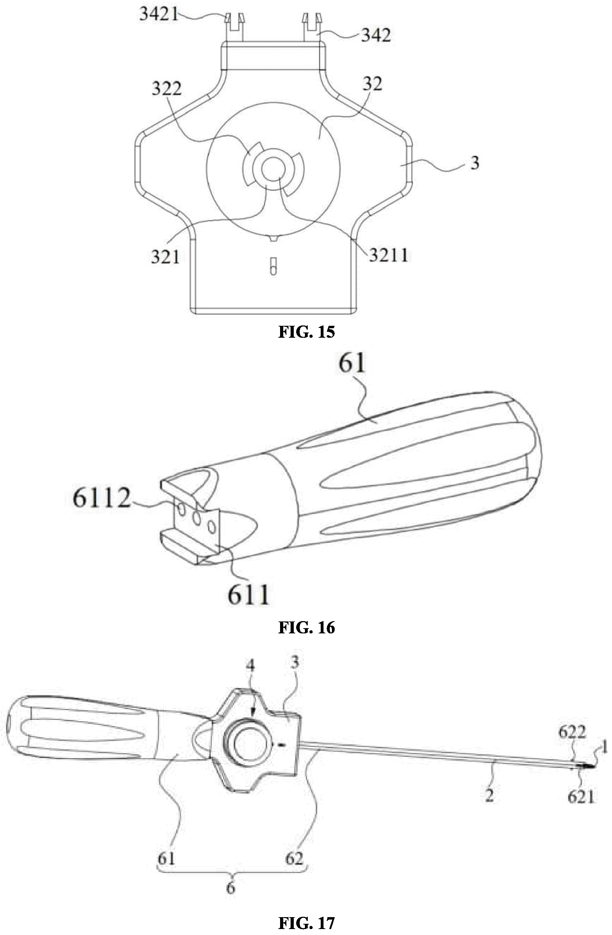

As shown in FIG. 2 and FIG. 17, the inserter 6 includes an insertion rod 62, two guide columns 622 are provided oppositely on the circumference of the insertion rod 62, and guide slots 311 are correspondingly provided on two opposite sides of the insertion cavity 31. When the insertion rod 62 enters from one end of the insertion cavity 31, two guide columns 622 and two guide slots 311 mate in one-to-one correspondence to provide guidance for the insertion rod 62. An end of the insertion rod 62 is provided with an insertion portion 621, and the insertion portion 621 is in insertion engagement with the anchor 1. For example, the insertion portion 621 is adoped as a structure engaging with the tail structure of the anchor 1. For example, the tail of a boss anchor is in a hexagonal pyramid shape, and the insertion portion 621 is adoped as an internal hole head, i.e., a hexagonal hole engaging with the hexagonal pyramid. The internal hole of the internal hole anchor is a hexagonal hole, and the insertion portion 621 is adopted as a prismatic head, i.e., a hexagonal prism engaging with the hexagonal hole. Two opposite sides of the hexagonal prism are provided with clearance slots, and two ends of the suture 2 pass through the clearance slots and extend out from the tail of the internal hole anchor 1. Alternatively, the insertion portion 621 is adopted as a fork-shaped head. When the anchor 1 is a full suture anchor, after the fork-shaped head clamps the flat structure in the middle of the full suture anchor, the insertion rod 62 applies a force to the full suture anchor, and the full suture anchor deforms and is V-shaped when being pushed out of the carrier seat 3.

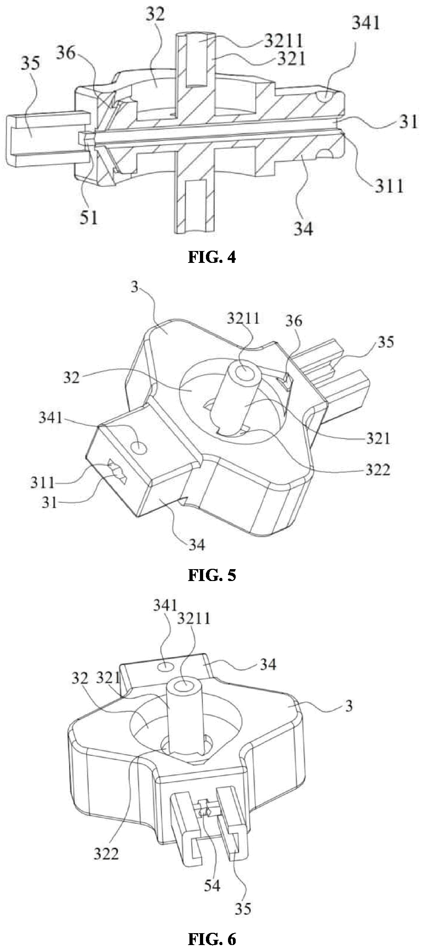

In one embodiment, as shown in FIG. 2 and FIG. 6, the suture anchor storage module further includes a clamping assembly 5, and the clamping assembly 5 is connected to the carrier seat 3; the clamping assembly 5 includes a first clamping member 51 and a second clamping member 52 arranged symmetrically, and the first clamping member 51 and the second clamping member 52 are oppositely disposed to form the clamping hole 54.

In this embodiment, the first clamping member 51 and the second clamping member 52 are each adopted as a clamping block, and the clamping hole 54 is formed by oppositely disposing two clamping blocks arranged at an end of the insertion cavity 31. The ends of the clamping blocks that are close to each other are adopted to engage with the shape of the anchor 1, such as adopted as V-shaped grooves, and the V-shaped grooves are engaged with the periphery of the anchor 1 to clamp the anchor 1.

Two clamping blocks and the carrier seat 3 are adopted as a one-piece structure. After the insertion portion 621 of the insertion rod 62 is inserted into the anchor 1, the inserter 6 continues to push the anchor 1, and two clamping blocks may be destroyed under a pushing force, so that the anchor 1 breaks from the clamping hole 54 and is pushed out of the carrier seat 3.

In this embodiment, the clamping blocks and the carrier seat 3 are integrally injection-molded, and the connections between the clamping blocks and the carrier seat 3 are weak connections and may be cut off under a pushing force.

Further, as shown in FIG. 5 and FIG. 6, two opposite extension slots 35 are provided at the end of the carrier seat 3 near the clamping blocks, two extension slots 35 are located on the sides of two clamping blocks away from each other and extend reversely away from the insertion cavity 31, and center lines of two extension slots 35 are on the same straight line as a center line of the insertion cavity 31 to ensure that an axis of the anchor 1 is on the same straight line as the center line of the insertion cavity 31, thereby ensuring that the insertion rod 62 can be accurately engaged with the anchor 1 when entering the insertion cavity 31 for smooth assembly.

A mounting groove 32 is provided on the side of the carrier seat 3, the mounting shaft 321 is arranged in the mounting groove 32, and the winding and tensioning assembly 4 is located in the mounting groove 32 after being sleeved on the mounting shaft 321. Through the mounting groove 32, the winding and tensioning assembly 4 is at least partially located in the installation slot 32, which not only reduces the volume of the suture anchor storage module, but also limits the winding and tensioning assembly 4 to some extent.

As shown in FIG. 4, a communication cavity 36 is provided between the insertion cavity 31 and the mounting groove 32. After two clamping blocks clamp the anchor 1, the suture 2 passing through the anchor 1 enters the mounting groove 32 through the communication cavity 36 from the end of the insertion cavity 31 near the clamping hole 54, and is wound on the winding and tensioning assembly 4.

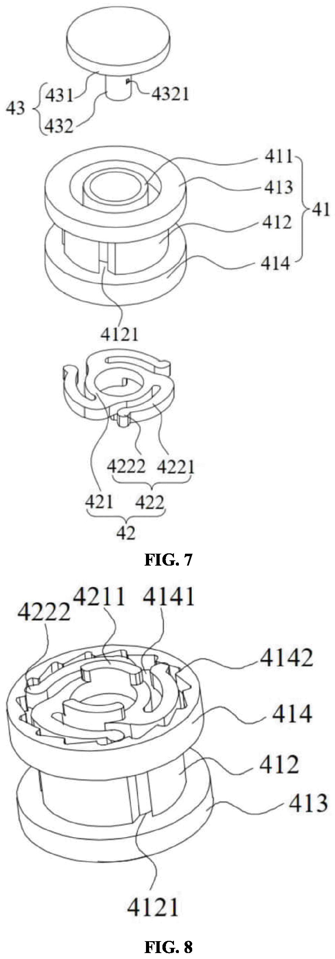

In one embodiment, as shown in FIG. 6 to FIG. 8, the winding and tensioning assembly 4 includes a winding reel 41 and a damping member 42, the winding reel 41 is sleeved on the mounting shaft 321 and rotatably connected to the mounting shaft 321, the bottom of the winding reel 41 is provided with a concave cavity 4141, the circumference of the concave cavity 4141 is adopted as an internal ratchet structure 4142, the damping member 42 is fixed on a side of the mounting groove 32 and located in the internal ratchet structure 4142, the damping member 42 includes an elastic pawl 422, the winding reel 41 rotates, and the elastic pawl 422 cooperates with the internal ratchet structure 4142 to provide rotational resistance to the winding reel 41. When the winding reel 41 rotates clockwise, the elastic pawl 422 forms a stepping motion with the internal ratchet structure 4142. When the winding reel 41 rotates relative to the mounting shaft 321, the internal ratchet structure 4142 compresses the elastic pawl 422, so that the winding reel 41 has certain rotational resistance, and the rotational resistance keeps the suture 2 in a tight state when the winding reel 41 rotates.

Specifically, the winding reel 41 includes an inner cylinder 411 and an outer cylinder 412 arranged coaxially, the inner cylinder 411 is sleeved on the mounting shaft 321, the outer cylinder 412 is provided with opposite suture retaining slots 4121, the suture 2 is wound on the outer cylinder 412, and a tail end of the suture 2 passes through the opposite suture retaining slots 4121 and is fixed. Two groups of suture retaining slots 4121 are provided, and two suture retaining slots 4121 in each group are arranged oppositely. After the suture 2 passes through the communication cavity 36 and enters the mounting groove 32, the suture is wound from the bottom to top of the outer cylinder 412, and the tail of the suture 2 passes through two opposite suture retaining slots 4121 in one group and then enters two opposite suture retaining slots 4121 in the other group for fixation.

In one embodiment, the winding and tensioning assembly 4 further includes a compression member 43, the compression member 43 includes a gland 431 and a connection column 432, the mounting shaft 321 is provided with a central hole 3211, the connection column 432 is engaged with the central hole 3211, and the winding reel 41 is rotatably arranged between the gland 431 and the carrier seat 3. Two protrusions 4321 are arranged oppositely in the circumferential direction of the connection column 432, and two protrusions 4321 are in clamping fit with an inner wall of the central hole 3211 to fix the compression member 43. The winding reel 41 rotates between the gland 431 and the bottom of the mounting groove 32.

Specifically, a limit top plate 413 and a limit bottom plate 414 are provided at two ends of the winding reel 41 respectively, the gland 431 is in limiting engagement with the limit top plate 413, and the concave cavity 4141 is arranged on the side of the limit bottom plate 414 away from the limit top plate 413. The limit top plate 413 and the limit bottom plate 414 are adopted to prevent the suture 2 from falling off during the rotation of the winding reel 41, and to limit the suture 2. Meanwhile, the gland 431 is located above the limit top plate 413, and the gland 431 can limit the winding reel 41 from flying out from an upper end of the mounting shaft 321 during rotation. The damping member 42 is arranged in a closed space formed by the concave cavity 4141 and the bottom of the mounting groove 32.

In one embodiment, the bottom of the mounting groove 32 is provided with a retaining groove 322, the bottom of the damping member 42 is provided with a clamping block 4211, and the clamping block 4211 is engaged with the retaining groove 322 to fix the damping member 42 and the carrier seat 3.

Specifically, the damping member 42 further includes a mounting ring 421, and the mounting ring 421 is sleeved on the mounting shaft 321 and fixedly connected to the carrier seat 3. The clamping block 4211 is arranged at a bottom of the mounting ring 421. Two clamping blocks 4211 are oppositely disposed, two retaining grooves 322 are correspondingly disposed, and two clamping blocks 4211 are arranged in two retaining grooves 322 in one-to-one correspondence to fix the damping member 42 to the bottom of the mounting groove 32.

A plurality of elastic pawls 422 are provided at intervals on the periphery of the mounting ring 421, one ends of the elastic pawls 422 are connected to the mounting ring 421, the other ends of the elastic pawls are bent into clamping rings 4221 spaced apart from the periphery of the mounting ring 421, a free end of the clamping ring 4221 is provided with a clamping protrusion 4222, and the clamping protrusion 4222 elastically abuts against the internal ratchet structure 4142. The clamping rings 4221 are spaced apart from the mounting ring 421, so that when the clamping protrusions 4222 are compressed by the internal ratchet structure 4142, the clamping rings 4221 have certain deformation space.

The internal ratchet structure 4142 includes a plurality of chutes connected sequentially, the chute includes an inclined edge and a bottom edge, the end of the inclined edge away from the bottom edge gradually inclines away from the center of a circle and extends to the bottom edge, the clamping protrusion 4222 includes a first arc abutting against the inclined edge and a second arc abutting against the bottom edge, and the second arc inclines downward from the first arc towards the center of the circle, so that when the winding reel 41 is subjected to maximum resistance, the bottom edge can break from the restriction of the clamping protrusion 4222 under the rotational force of the winding reel 41, the winding reel 41 continues to rotate, and the clamping protrusion 4222 abuts against the next chute. When the winding reel 41 rotates clockwise, the first arc abuts against the end of the inclined edge away from the bottom edge. As the winding reel 41 rotates, the clamping ring 4221 gradually moves away from the center of the circle until the second arc abuts against the bottom edge, and the winding reel 41 is subjected to the maximum resistance. The winding reel 41 continues to rotate. Under the action of rotational force, the bottom edge gradually disengages from abutment against the second arc, and the inclined edge of the next chute abuts against the first arc to start next repetitive motion. When the winding reel 41 rotates counterclockwise, the clamping protrusion 4222 of the clamping ring 4221 is subjected to an opposite force, and passes through the end of the inclined edge away from the bottom edge before the next repetitive motion.

Two winding and tensioning assemblies 4 are provided. The winding and tensioning assemblies 4 are symmetrically arranged on two sides of the carrier seat 3. One end of the suture 2 passing through the anchor 1 is wound on one winding and tensioning assembly 4, and the other end is wound on the other winding and tensioning assembly 4.

In this embodiment, with continued reference to FIG. 4, mounting grooves 32 are provided on two opposite sides of the carrier seat 3, a winding and tensioning assembly 4 is mounted in each mounting groove 32, and a communication cavity 36 is provided on each of two opposite sides of the insertion cavity 31. After the suture 2 passes through the anchor 1, one end of the suture passes through one communication cavity 36 to enter one mounting groove 32 and is wound on the winding reel 41 in the mounting groove 32; and the other end of the suture passes through the other communication cavity 36 to enter the other mounting groove 32 and is wound on the winding reel 41 in the mounting groove 32.

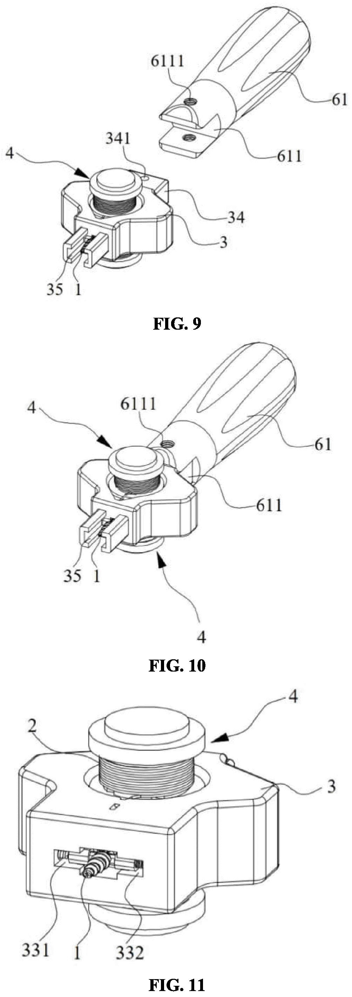

As shown in FIG. 9 and FIG. 10, the inserter 6 further includes a handle 61, the insertion rod 62 is connected to the handle 61, and an insertion portion 621 is provided at the end of the insertion rod 62 away from the handle 61. The insertion rod 62 is a metal rod, and the handle 61 is made of plastic. The metal rod may be injection-molded into the handle 61 or detachably connected to the handle 61.

After the insertion rod 62 of the inserter 6 enters the insertion cavity 31 to engage with the anchor 1 and push the anchor 1 out of the carrier seat 3, the inserter 6 is fixed to the carrier seat 3. The end of the handle 61 near the insertion rod 62 is provided with a clamping groove 611, the end of the carrier seat 3 away from the clamping hole 54 is adopted as a clamping end 34, the clamping end 34 cooperates with the clamping groove 611, the clamping end 34 is provided with a first fixing structure, the clamping groove 611 is provided with a second fixing structure, and the first fixing structure cooperates with the second fixing structure to fix the carrier seat 3 with the handle 61.

In one embodiment, the first fixing structure includes fixing grooves 341, and the fixing grooves 341 are arranged on two opposite sides of the clamping end 34; the second fixing structure includes connection holes 6111, and the connection holes 6111 are arranged on two opposite side walls of the clamping groove 611.

Specifically, the connection hole 6111 is adopted as a threaded hole for mounting a positioning bead. The positioning bead is also known as a ball plunger or a spring plunger, and is a load device composed of a shell, a spring, and a ball or cylinder. When the clamping end 34 enters the clamping groove 611 and moves in place, the ball of the positioning bead slides into the fixing groove 341 to limit the clamping end 34, thereby achieving fixation.

In other embodiments, a fastening screw may be mounted in the connection hole 6111 to fix the clamping end 34.

After the carrier seat 3 is fixed to the handle 61, the anchor 1 has been completely pushed out of the carrier seat 3. At this time, the anchor 1 may be implanted into the bone by twisting or tapping the handle 61. After the anchor 1 is implanted into the bone, the handle 61 moves in a direction opposite to the implantation direction, the carrier seat 3 moves with the insertion rod 62 away from the anchor 1, the insertion portion 621 gradually separates from the anchor 1, the suture 2 drives the winding reel 41 to rotate, and the suture 2 is gradually released. During the release of the suture 2, the clamping protrusions 4222 of the elastic pawls 422 of the damping member 42 cooperate with the chutes of the internal ratchet structure 4142 to provide certain resistance to the rotation of the winding reel 41, enabling the winding reel 41 to move step by step, thereby ensuring that the suture 2 remains tight until the tail end of suture 2 breaks from the suture retaining slots 4121 and slides out of the carrier seat 3. Then, the suture 2 is completely released. After the release of the suture 2 is completed, the carrier seat 3 is removed from the inserter 6. The inserter 6 can further be assembled with the next anchor storage module to complete the implantation of the next anchor 1, achieving reuse of the inserter 6.

### Second embodiment:

As shown in FIG. 11 to FIG. 14, this embodiment provides a separable modular suture anchor implant system and a suture anchor storage module applied to the separable modular suture anchor implant system. The structure of the suture anchor storage module is substantially the same as that of the suture anchor storage module provided in the first embodiment, except for the specific structure of the clamping assembly 5 and the matching and fixing structure of the carrier seat 3 and the handle 61.

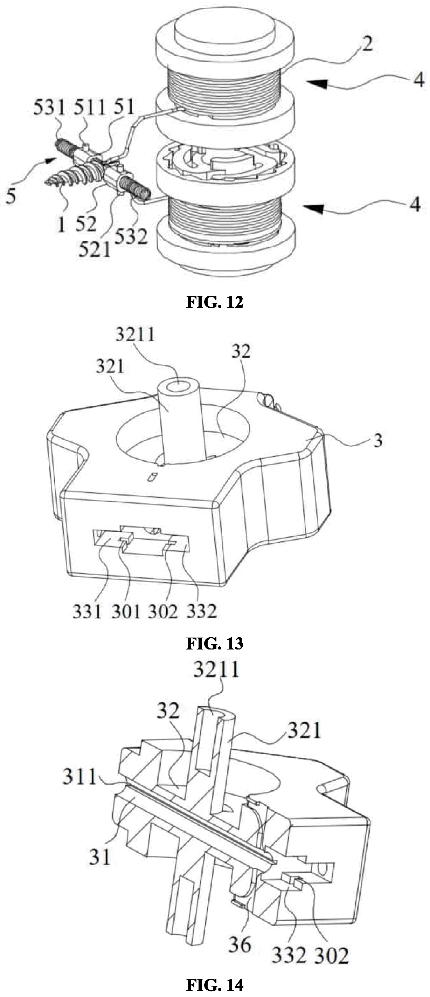

Specifically, as shown in FIG. 12, the ends of the first clamping member 51 and the second clamping member 52 that are close to each other are adopted as V-shaped grooves, and two V-shaped grooves are oppositely disposed to form the clamping hole 54 for clamping the circumference of the anchor 1.

The clamping assembly 5 further includes two elastic members, i.e., a first elastic member 531 and a second elastic member 532. The first elastic member 531 is arranged at the end of the first clamping member 51 away from the second clamping member 52, and the first elastic member 531 is used to elastically connect the first clamping member 51 to the carrier seat 3; the second elastic member 532 is arranged at the end of the second clamping member 52 away from the first clamping member 51; and the second elastic member 532 is used to elastically connect the second clamping member 52 to the carrier seat 3.

With continued reference to FIG. 11 to FIG. 13, the end of the carrier seat 3 away from the clamping end 34 is provided with a first mounting cavity 331 and a second mounting cavity 332 oppositely, the first clamping member 51 and the first elastic member 531 are mounted in the first mounting cavity 331, the second clamping member 52 and the second elastic member 532 are mounted in the second mounting cavity 332, the bottom of the first mounting cavity 331 is provided with a first mounting hole, and the bottom of the second mounting cavity 332 is provided with a second mounting hole. The ends of the first clamping member 51 and the second clamping member 52 that are away from each other are provided with spring cavities, i.e., a first spring cavity and a second spring cavity. One end of the first elastic member 531 is arranged in the first spring cavity, and the other end of the first elastic member 531 is fixed in the first mounting hole. One end of the second elastic member 532 is arranged in the second spring cavity, and the other end is fixed in the second mounting hole.

Both the first clamping member 51 and the second clamping member 52 are slidably arranged in the carrier seat 3; sliding grooves are provided in the carrier seat 3, sliding columns are provided on both the first clamping member 51 and the second clamping member 52, the sliding columns are slidably arranged in the sliding grooves, and the sliding grooves extend along an elastic expansion direction of the elastic member. The sliding columns cooperate with the sliding grooves to limit the movement positions of the first clamping member 51 and the second clamping member 52.

Specifically, with continued reference to FIG. 12 and FIG. 13, sliding columns, i.e., a first sliding column 511 and a second sliding column 521, are provided on two opposite sides of each of the first clamping member 51 and the second clamping member 52, the sliding groove includes a first sliding groove 301 and a second sliding groove 302, and the first sliding grooves 301 are provided on two opposite sides of the first mounting cavity 331; the second sliding grooves 302 are provided on two opposite sides of the second mounting cavity 332, the first sliding columns 511 cooperate with the first sliding grooves 301, and the first sliding grooves 301 are used to limit the movement distance of the first clamping member 51; the second sliding columns 521 cooperate with the second sliding grooves 302, and the second sliding grooves 302 are used to limit the movement distance of the second clamping member 52.

After the insertion rod 62 enters the insertion cavity 31 and is inserted and fixed to the anchor 1, the insertion rod continues to push the anchor 1. Under the pushing force of the insertion rod 62, the first clamping member 51 compresses the first elastic member 531, and the second clamping member 52 compresses the second elastic member 532, so that the first clamping member 51 and the second clamping member 52 move away from each other, and the anchor 1 breaks from the clamping hole 54. When the anchor 1 completely breaks from the clamping hole 54, the first clamping member 51 and the second clamping member 52 jointly clamp two opposite sides of the insertion rod 62.

As shown in FIG. 15 to FIG. 18, the first fixing structure at the clamping end 34 of the carrier seat 3 includes a fixing nail 342, the fixing nail 342 is arranged at the end of the clamping end 34 away from the clamping hole 54, and an end of the fixing nail 342 is provided with a hook 3421. The second fixing structure at the clamping groove 611 of the handle 61 includes an insertion hole 6112 and a fixing groove 6113, the insertion hole 6112 is arranged at the bottom of the clamping groove 611, and the fixing groove 6113 is arranged at the end of the insertion hole 6112 away from the clamping groove 611. After the insertion rod 62 enters the insertion cavity 31 to assemble with the anchor 1 and pushes the anchor 1 out of the carrier seat 3, the insertion rod continues to move until the clamping end 34 of the carrier seat 3 enters the clamping groove 611, and the fixing nail 342 enters the insertion hole 6112. During the movement of the fixing nail 342 in the insertion hole 6112, the wall of the insertion hole 6112 compresses the hook 3421. After the hook 3421 moves out of the insertion hole 6112, the hook 3421 abuts against the connection between the fixing groove 6113 and the insertion hole 6112, thereby achieving fixed connection between the carrier seat 3 and the handle 61.

### Third embodiment:

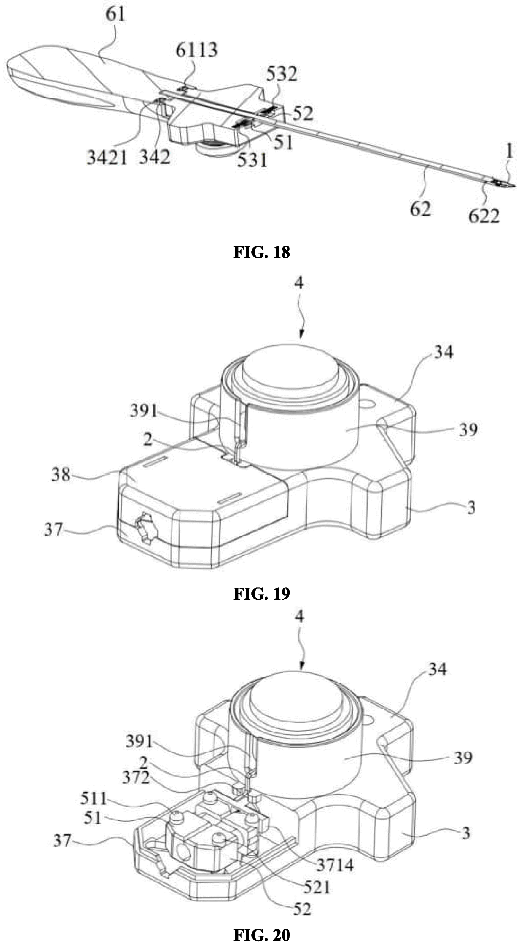

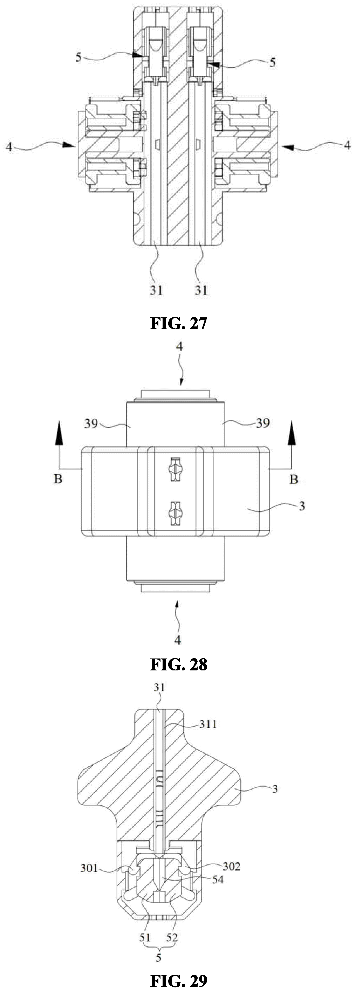

As shown in FIG. 19 to FIG. 29, this embodiment provides a separable modular suture anchor implant system and a suture anchor storage module applied to the separable modular suture anchor implant system. The structure of the suture anchor storage module is substantially the same as that of the suture anchor storage module provided in the first embodiment, except for the quantity of the winding and tensioning assembly 4 and the structure of the clamping assembly 5.

Specifically, as shown in FIG. 19 to FIG. 21, one winding and tensioning assembly 4 is provided, the winding and tensioning assembly 4 is arranged on one side of the carrier seat 3, and two ends of the suture 2 passing through the anchor 1 are each wound on the winding and tensioning assembly 4. That is, the winding and tensioning assembly 4 is arranged on a single side of the carrier seat 3, and two ends of the suture 2 passing through the anchor 1 are each wound on the same winding reel 41. When the anchor 1 moves under the pushing force of the insertion rod 62, the suture 2 can be released simultaneously, ensuring uniform release of the suture 2 at two ends of the anchor 1.

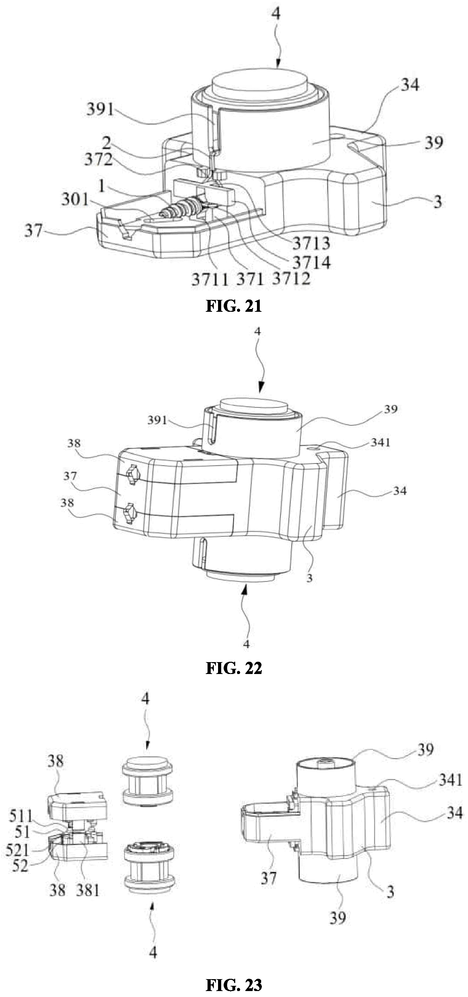

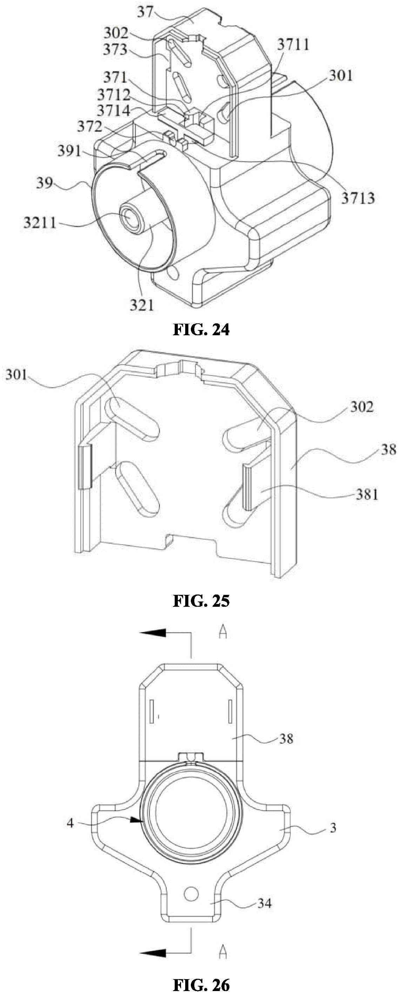

As shown in FIG. 23 and FIG. 24, the end of the carrier seat 3 away from the clamping end 34 is provided with a mounting bottom shell 37 and an upper cover 38, and the upper cover 38 is fastened to the mounting bottom shell 37 to form an outlet at the end away from the insertion cavity 31, allowing the insertion rod 62 to push out the anchor 1. Specifically, the mounting bottom shell 37 is fixed to the upper cover 38 by snap fit, clamping bars 381 extending down are provided on two opposite sides of the upper cover 38, the end of the clamping bar 381 away from the upper cover 38 is adopted as a hook end, bayonets 373 are provided on two opposite sides of the mounting bottom shell 37, and the hook ends cooperate with the bayonets 373 for clamping.

With continued reference to FIG. 21, in order to implement concentrated winding of the suture 2 on the same winding reel 41 from two sides of anchor 1, a guide assembly is provided on the bottom shell 37, the guide assembly includes a guide block 371 and a suture collection groove 372, the suture collection groove 372 is arranged above the guide block 371, the guide block 371 is provided with a through hole 3711 connecting the insertion cavity 31 with the clamping hole 54, a clearance space is reserved between the through hole 3711 and the clamping hole 54, guide grooves 3712 are provided at two ends of the side of the through hole 3711 near the clearance space, a blocking bar 3714 is provided above the guide groove 3712, two guide inclined surfaces 3713 are symmetrically provided behind the blocking bar 3714, and the suture collection groove 372 is located above two guide inclined surfaces 3713. Two ends of the suture 2 passing through the anchor 1 are threaded through the clearance space, then through the guide grooves 3712 around to the back of the blocking bars 3714, and to the suture collection groove 372 along the guide inclined surfaces 3713, and two ends of the suture 2 are concentrated in the suture collection groove 372.

A mounting cylinder 39 is further provided on the mounting groove 32 of the carrier seat 3, an upper end of the mounting cylinder 39 is open, the winding and tensioning assembly 4 is located in the mounting cylinder 39, and the mounting cylinder 39 is provided with a passing groove 391 corresponding to the suture collection groove 372. Two ends of the suture 2, after concentrated in the suture collection groove 372, jointly pass through the passing groove 391 to enter the mounting cylinder 39, and are each wound on the winding reel 41 in the same direction.

With continued reference to FIG. 20, the first clamping member 51 is a first clamping jaw, the second clamping member 52 is a second clamping jaw, the clamping assembly 5 further includes an elastic member, one elastic member is provided, and the first clamping member 51 and the second clamping member 52 are connected by the elastic member. The first clamping jaw and the second clamping jaw are connected by the elastic member, the entire anchor 1 is gripped in the clamping hole 54 formed by oppositely disposing the first clamping jaw and the second clamping jaw, and the anchor 1 is tightly clamped by means of the elasticity of the elastic member. In two ends of the clamping hole 54, a limit plane is provided at the end close to the insertion cavity 31, and a limit inclined plane is provided at the end away from the insertion cavity 31. The limit plane and the limit inclined plane jointly limit the movement of the anchor 1 in the clamping hole 54, thereby ensuring that the insertion portion 621 can be smoothly inserted into the anchor 1.

Specifically, a semi-annular groove is provided on the circumference of each of the first clamping jaw and the second clamping jaw, two semi-annular grooves are oppositely disposed to form a limit groove, the elastic member is adopted as an elastic band, and the elastic band is placed in the limit groove to fix the first clamping jaw and the second clamping jaw together for storing the anchor 1.

Both the first clamping jaw and the second clamping jaw are slidably arranged in the carrier seat 3. The first clamping jaw and the second clamping jaw can be driven to move away from each other under the pushing force of the insertion rod 62, the ends of the first clamping jaw and the second clamping jaw away from the insertion cavity 31 open, and the insertion rod 62 pushes the anchor 1 out of the clamping hole 54.

As shown in FIG. 23 and FIG. 24, in order to ensure the stability of movement of the first clamping jaw and the second clamping jaw under the force, first sliding columns 511 are provided on two opposite sides of the first clamping jaw, first sliding grooves 301 are correspondingly provided on the mounting bottom shell 37 and the upper cover 38, and the first sliding columns 511 cooperate with the first sliding grooves 301; second sliding columns 521 are provided on two opposite sides of the second clamping jaw, second sliding grooves 302 are correspondingly provided on the mounting bottom shell 37 and the upper cover 38, and the second sliding columns 521 cooperate with the second sliding grooves 302. The first sliding grooves 301 extend away from the first clamping jaw and the insertion cavity 31, and the second sliding grooves 302 extend away from the second clamping jaw and the insertion cavity 31, to ensure that the opening angle of the ends of the first clamping jaw and the second clamping jaw near the insertion cavity 31 is smaller than the opening angle of the ends away from the insertion cavity 31.

In this embodiment, two first sliding columns 511 are provided on two opposite sides of the first clamping jaw, two first sliding grooves 301 are correspondingly provided on the mounting bottom shell 37 and the upper cover 38, and the angle between the first sliding groove 301 near the insertion cavity 31 and the symmetrical line of the first clamping jaw and the second clamping jaw is smaller than that between the first sliding groove 301 away from the insertion cavity 31 and the symmetrical line of the first clamping jaw and the second clamping jaw. Two second sliding columns 521 are provided on two opposite sides of the second clamping jaw, two second sliding grooves 302 are correspondingly provided on the mounting bottom shell 37 and the upper cover 38, and the angle between the second sliding groove 302 near the insertion cavity 31 and the symmetrical line of the first clamping jaw and the second clamping jaw is smaller than that between the second sliding groove 302 away from the insertion cavity 31 and the symmetrical line of the first clamping jaw and the second clamping jaw. Such arrangement can better control the movement path of the first clamping jaw and the second clamping jaw, and ensure the opening angle of the ends of the first clamping jaw and the second clamping jaw away from the insertion cavity 31.

In other embodiments, the elastic member may be adopted as an elastic ring, the elastic ring surrounds the first sliding column 511 and the second sliding column 521 located on the same side, the first sliding column 511 and the second sliding column 521 each include a column body and a sliding head, the outer diameter of the sliding head is greater than that of the column body, the sliding head of the first sliding column 511 is slidably arranged in the first sliding groove 301, the sliding head of the second sliding column 521 is slidably arranged in the second sliding groove 302, and the elastic ring is clamped between the sliding head and the side wall of the clamping jaw.

In actual surgery, a plurality of anchors 1 or various anchors 1 may be required. In order to save costs, the winding and tensioning assembly 4 is arranged on the single side of the carrier seat 3, as shown in FIG. 22 to FIG. 29, the suture anchor storage module may be provided with double anchors and double winding and tensioning assemblies, two insertion cavities 31 and two clamping holes 54 are provided, and two insertion cavities 31 and two clamping holes 54 correspond one to one. That is, two insertion cavities 31 and two clamping holes 54 are provided in one carrier seat 3, one anchor 1 is gripped in the clamping hole 54 corresponding to each insertion cavity 31, one winding and tensioning assembly 4 is provided on each of two sides of the carrier seat 3, and the suture 2 for each anchor 1 is wound on one winding and tensioning assembly 4.

The suture anchor storage module with double anchors and double winding and tensioning assemblies, provided in this embodiment, can store not only two anchors 1 of the same type but also two anchors 1 of different types, that is, one suture anchor storage module stores two types of anchors 1, facilitating preoperative material preparation for surgeries requiring diverse types of anchors 1.

Described above are merely the preferred embodiments of the present application. For a person of ordinary skill in the art, modifications may be made to the specific implementation and application scope based on the idea of the present application, and the content of this specification should not be understood as limiting the present application.

## Claims

1. A suture anchor storage module, comprising:
an anchor (1);
a suture (2) threaded through the anchor (1);
a carrier seat (3), wherein the carrier seat (3) is internally provided with an insertion cavity (31) and a clamping hole (54), and the insertion cavity (31) is disposed through two opposite ends of the carrier seat (3) and is for allowing an inserter (6) to pass through; the clamping hole (54) is in communication with the insertion cavity (31) to clamp the anchor (1); and a mounting shaft (321) is provided on a side of the carrier seat (3); and
a winding and tensioning assembly (4), wherein the winding and tensioning assembly (4) is rotatably connected to the mounting shaft (321), the suture (2) is wound on the winding and tensioning assembly (4), and the winding and tensioning assembly (4) is configured to rotate to release the suture (2).

2. The suture anchor storage module according to claim 1, wherein the winding and tensioning assembly (4) comprises a winding reel (41) and a damping member (42), the winding reel (41) is sleeved on the mounting shaft (321) and rotatably connected to the mounting shaft (321), a bottom of the winding reel (41) is provided with a concave cavity (4141), a circumference of the concave cavity (4141) is configured as an internal ratchet structure (4142), the damping member (42) is fixed on a side of the carrier seat (3) and located in the internal ratchet structure (4142), the damping member (42) comprises elastic pawls (422), the winding reel (41) is configured to rotate, and each of the elastic pawls (422) is configured to engage with the internal ratchet structure (4142) to provide rotational resistance to the winding reel (41).

3. The suture anchor storage module according to claim 2, wherein the damping member (42) further comprises a mounting ring (421), and the mounting ring (421) is sleeved on the mounting shaft (321) and fixedly connected to the carrier seat (3); a plurality of the elastic pawls (422) are provided at intervals on a periphery of the mounting ring (421), one end of each of the elastic pawls (422) is connected to the mounting ring (421), the other end of each of the elastic pawls is bent into a clamping ring (4221), the clamping rings are spaced apart from the periphery of the mounting ring (421), a free end of the clamping ring (4221) is provided with a clamping protrusion (4222), and the clamping protrusion (4222) elastically abuts against the internal ratchet structure (4142).

4. The suture anchor storage module according to claim 2, wherein the winding reel (41) comprises an inner cylinder (411) and an outer cylinder (412) arranged coaxially, the inner cylinder (411) is sleeved on the mounting shaft (321), the outer cylinder (412) is provided with opposite suture retaining slots (4121), the suture (2) is wound on the outer cylinder (412), and a tail end of the suture (2) is fixed by passing through the opposite suture retaining slots (4121).

5. The suture anchor storage module according to claim 2, wherein the winding and tensioning assembly (4) further comprises a compression member (43), the compression member (43) comprises a gland (431) and a connection column (432), the mounting shaft (321) is provided with a central hole (3211), the connection column (432) is engaged with the central hole (3211), and the winding reel (41) is rotatably arranged between the gland (431) and the carrier seat (3).

6. The suture anchor storage module according to claim 5, wherein a limit top plate (413) and a limit bottom plate (414) are provided at two ends of the winding reel (41) respectively, the gland (431) is in limiting engagement with the limit top plate (413), and the concave cavity (4141) is arranged on a side of the limit bottom plate (414) away from the limit top plate (413).

7. The suture anchor storage module according to claim 2, wherein a mounting groove (32) is provided on the side of the carrier seat (3), the mounting shaft (321) is arranged in the mounting groove (32), a bottom of the mounting groove (32) is provided with a retaining groove (322), a bottom of the damping member (42) is provided with a clamping block (4211), and the clamping block (4211) is engaged with the retaining groove (322).

8. The suture anchor storage module according to claim 1, wherein two winding and tensioning assemblies (4) are provided, the two winding and tensioning assemblies (4) are symmetrically arranged on two sides of the carrier seat (3), one end of the suture (2) passing through the anchor (1) is wound on one of the two winding and tensioning assembly (4), and the other end of the suture is wound on the other one of the two winding and tensioning assembly (4); or
one winding and tensioning assembly (4) is provided, the winding and tensioning assembly (4) is arranged on one side of the carrier seat (3), and two ends of the suture (2) passing through the anchor (1) are each wound on the winding and tensioning assembly (4).

9. The suture anchor storage module according to claim 1, wherein the suture anchor storage module further comprises a clamping assembly (5), and the clamping assembly (5) is connected to the carrier seat (3); the clamping assembly (5) comprises a first clamping member (51) and a second clamping member (52) arranged symmetrically, and the first clamping member (51) and the second clamping member (52) are oppositely disposed to form a clamping hole (54).

10. The suture anchor storage module according to claim 9, wherein the clamping assembly (5) further comprises an elastic member, one elastic member is provided, and the elastic member connects the first clamping member (51) and the second clamping member (52); or
two elastic members comprising a first elastic member (531) and a second elastic member (532) are provided, the first elastic member (531) is arranged at the end of the first clamping member (51) away from the second clamping member (52), and the first elastic member (531) is configured to elastically connect the first clamping member (51) to the carrier seat (3); the second elastic member (532) is arranged at the end of the second clamping member (52) away from the first clamping member (51); and the second elastic member (532) is configured to elastically connect the second clamping member (52) to the carrier seat (3).

11. The suture anchor storage module according to claim 10, wherein both the first clamping member (51) and the second clamping member (52) are slidably arranged in the carrier seat (3); a sliding groove is provided in the carrier seat (3), a sliding column is provided on each of the first clamping member (51) and the second clamping member (52), the sliding column is engaged with the sliding groove, and the sliding groove extends along an elastic expansion direction of the elastic member.

12. The suture anchor storage module according to claim 1, wherein two insertion cavities (31) and two clamping holes (54) are provided, and the two insertion cavities (31) and the two clamping holes (54) are correspondingly arranged.

13. The suture anchor storage module according to any one of claims 1-12, wherein the end of the carrier seat (3) away from the clamping hole (54) is configured as a clamping end (34), and the clamping end (34) is provided with a first fixing structure.

14. The suture anchor storage module according to claim 13, wherein the first fixing structure comprises fixing grooves (341), and the fixing grooves (341) are arranged on two opposite sides of the clamping end (34); or
the first fixing structure comprises a fixing nail (342), the fixing nail (342) is arranged at the end of the clamping end (34) away from the clamping hole (54), and an end of the fixing nail (342) is provided with a hook (3421).

15. A separable modular suture anchor implant system, comprising an inserter (6) and the suture anchor storage module according to any one of claims 1 to 14, wherein after the inserter (6) is inserted into the insertion cavity (31) and then into the anchor (1), and pushes the anchor (1) out of the carrier seat (3), the inserter (6) is fixed to the carrier seat (3).

16. The separable modular suture anchor implant system according to claim 15, wherein the inserter (6) comprises a handle (61) and an insertion rod (62), the insertion rod (62) is connected to the handle (61), an end of the insertion rod (62) away from the handle (61) is provided with an insertion portion (621), and the insertion portion (621) is in insertion engagement with the anchor (1).

17. The separable modular suture anchor implant system according to claim 16, wherein an end of the handle (61) close to the insertion rod (62) is provided with a clamping groove (611), and the clamping groove (611) is provided with a second fixing structure.

18. The separable modular suture anchor implant system according to claim 17, wherein the second fixing structure comprises connection holes (6111), and the connection holes (6111) are arranged on two opposite side walls of the clamping groove (611); or
the second fixing structure comprises an insertion hole (6112) and a fixing groove (6113), the insertion hole (6112) is arranged at a bottom of the clamping groove (611), and the fixing groove (6113) is arranged at an end of the insertion hole (6112) away from the clamping groove (611).
